# EUROPEAN PATENT SPECIFICATION

(11) **EP 0 843 578 B1**
(45) Date of publication and mention of the grant of the patent: **25.02.2004**
(21) Application number: 95941964.9
(22) Date of filing: 15.12.1995
(51) Int. Cl.: A61N 5/10, G21K 1/04, A61B 6/06

(54) **DEVICE FOR AUTOMATIC FINAL COLLIMATION OF IONIZING RADIATION**
VORRICHTUNG ZUR AUTOMATISCHEN ENDKOLLIMATION VON IONISIERENDEN STRAHLUNGEN
DISPOSITIF DE COLLIMATION FINALE AUTOMATIQUE DE RAYONS IONISANTS

(30) Priority: 19.12.1994 SE 9404405
(43) Date of publication of application: 27.05.1998
(73) Proprietor: ELEKTA AB, 103 93 Stockholm (SE)
(72) Inventor: BACKMAN, Per, S-583 47 Linköping (SE)
(74) Representative: Ellner, Lars O.
(86) International application number: PCT/SE1995/001523
(87) International publication number: WO 1996/019262

(56) References cited:
- EP-A- 0 498 438
- US-A- 4 514 859
- US-A- 5 216 255

## Description

### The technique's standpoint:

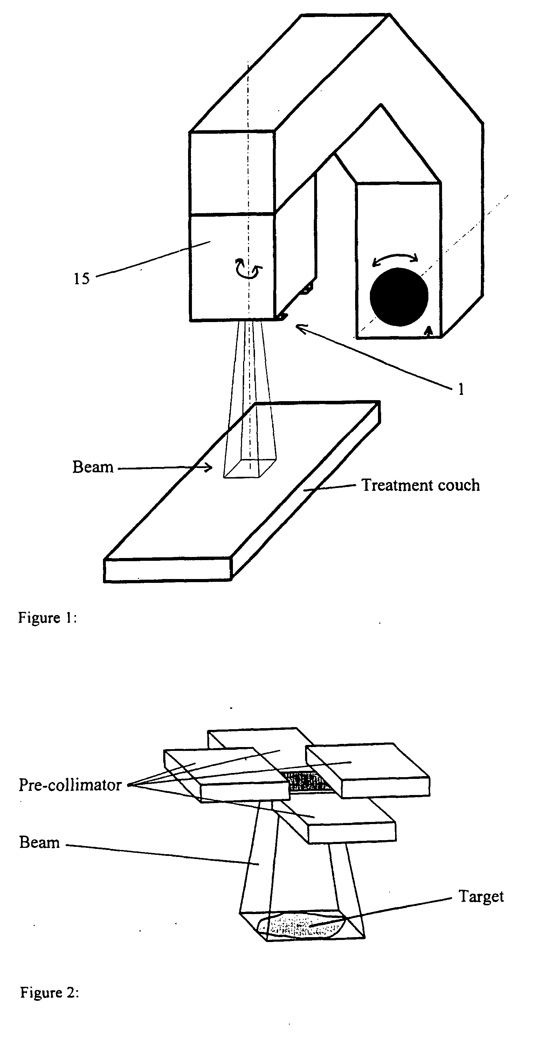

In radiation treatment of tumours in the human body using ionizing radiation produced in a linear accelerator, see figure 1, today 4 "stationary blocks" (pre-collimators) are used to screen off the beam to be square shaped. The size of the square depends on the target's outer contour, see figure 2.

To shape the beam more exact to the target (final collimating) today a number of devices are used. For example separate final collimators are moulded with a unique shape for each target. This is the most exact method but also the most time consuming. Another method is to place separate pieces of some material with high density in the beam field to shield off the beam to the shape of the target. At each treatment also the number of beam directions varies, mostly 6-8, which means that the final collimator's shape must be modified between each direction. All this is manual work and very time consuming.

When the collimation is not manual mostly multileaf-collimators are used, but these are often too complicated, large, heavy and too expensive for some applications. Multileaf-collimators' function can be compared to a profile template.

All the different types of final collimators are attached in a holder under the "stationary" collimator-blocks, see figure 1 (1). The manual collimators are detachable, and the multileaf collimator is fixed to the gantry. The stationary collimator blocks according to figure 2 are placed normally at pos. 15 in figure 1.

US-A-5 216 255 is directed to a system for applying radiation treatment according to an isodose contour. This contour is shaped by means of a collimator assembly consisting of four plates each having a separate drive unit. The entire collimator assembly can be rotated 90° as a unit.

EP-A-0 498 438 illustrates an X-ray diagnostic system that makes it possible to eliminate offsets in X-ray radiated fields. The system comprises four movable wings. Each of the wings can be moved in two directions and these wings can also be rotated together as a unit.

US-A-4 514 859 shows a collimator for an X-ray examination apparatus having four diaphragm plates each suspended by an associated linkage system. A common motor brings the plates in unison to move towards or away from the center of the beam in a rotational movement.

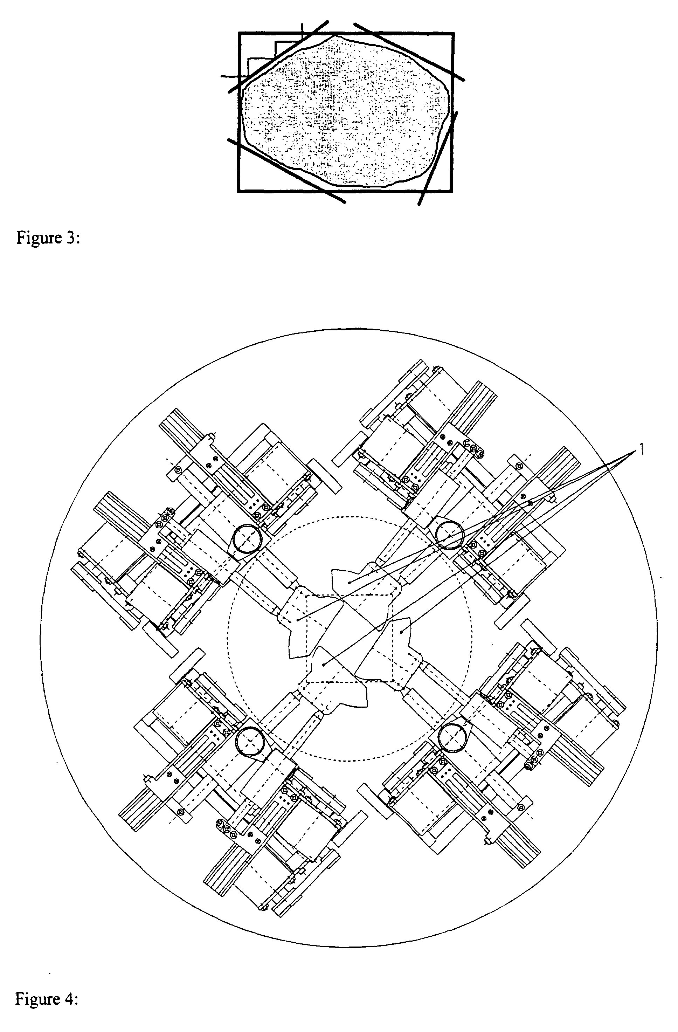

Estimations have been done which prove that you can gain conformity of the radiation to the target by "cutting off" the comers in the square shaped by the four above mentioned stationary collimator blocks, see figure 3.

The rather exact shape after the target, allowed by the multileaf collimators, which results in a very small size of radiated healthy can also be achieved by using the invention described below. When treating certain circular targets the multileaf-collimators give an inferior result than the described invention would give, see comparing geometry in the upper left comer in figure 3. (The multileaf profile has step form in the figure). A "cutting off" corners collimation is often used in the earlier described manual collimation with separate blocks. There is a request to automatize this procedure.

### The purpose of the invention and its most important features

The purpose is to achieve a device that in many treatment cases could replace the above described manual procedure. The purpose is to achieve a less expensive device than the multileaf collimators used today, with the same conformity of the radiation field. The most important feature of this device is that 4 collimator blocks are used as final collimator. Each of these blocks is used to "cut off" a comer of the square shaped radiation area made by the pre-collimator, see figure 3. The cutting off can be made for various sizes of the squares and in various angles.

### Description of the figures

- Figure **1**: Describes:
the principle for radiation when using linear
accelerator
- **2**: the principle of the pre-collimator
- **3**: the principle for "cutting off" comers with one example of a multileaf shape cut off
- **4**: the collimators (1) and driving mechanism (bottom view)
- **5**: detailed drawing of one collimator and its driving mechanism
- **6**: the collimator mounted in its housing (side view)
- **7**: drawing of the principle of the intervention of the beam by 2 collimators (side view)

### Description of the invention

The present invention provides a device to limit radiation as defined in claim 1.

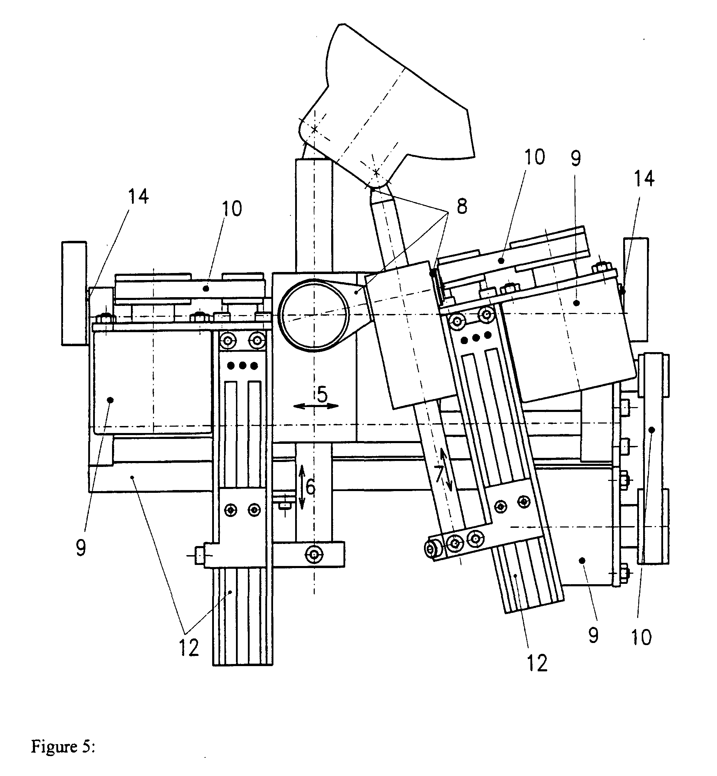

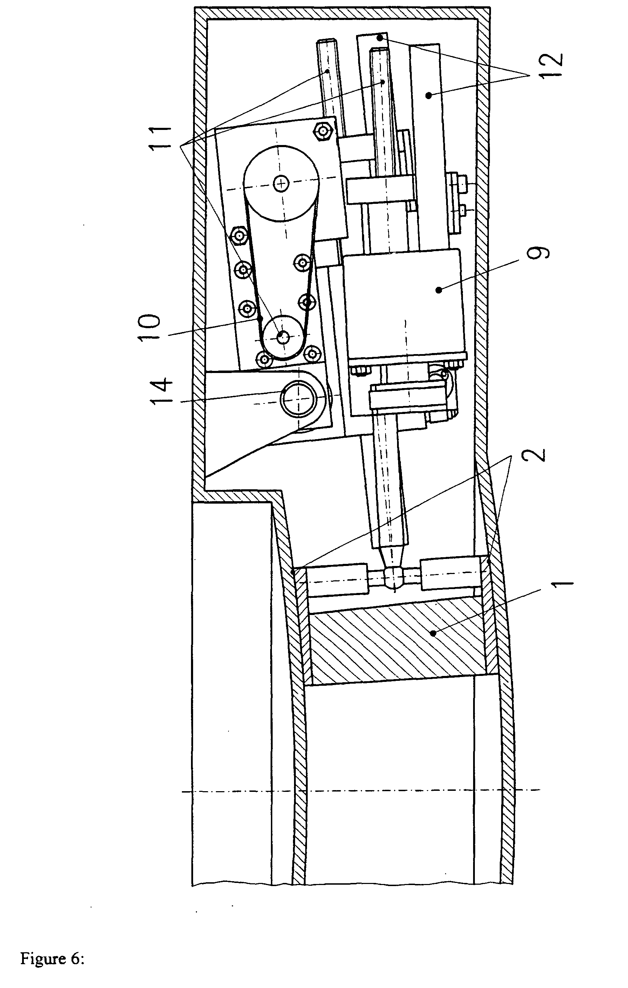

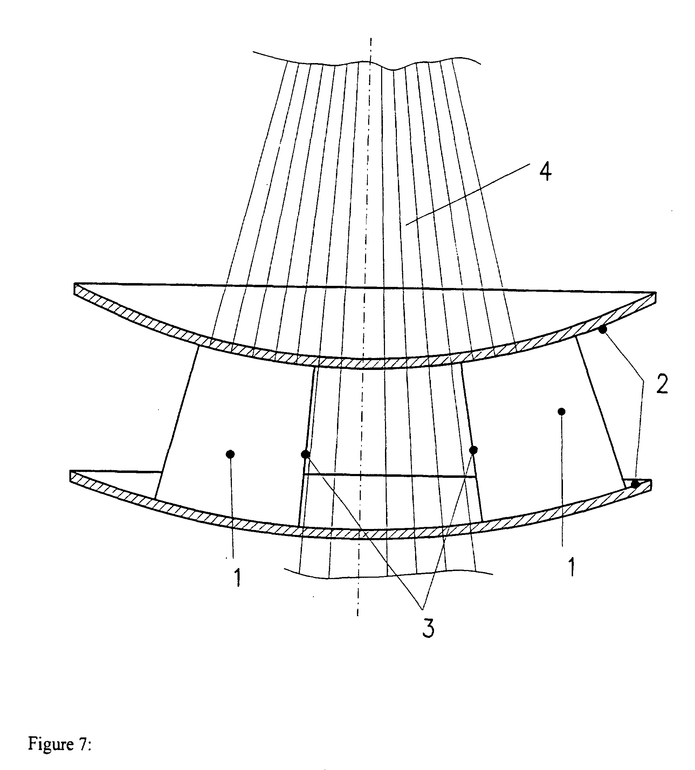

The arrangement consists of 4 identical units, see figure 4. There is one unit in each comer, in figure 5 only one unit is shown. The four movable comer blocks (1), made of high density material, like tungsten, are situated between two spherical surfaces (2) resulting in always keeping the edges of the blocks parallel (3) to the beam (4). The comer blocks' (1) geometry is such that all sides of the comer blocks (1) always are in the normal direction from the spherical surfaces (2) which they are controlled by. Three linear movements make the positioning of the blocks. One movement (5) is parallel with the moving area itself. The second linear movement works perpendicular to the first one (6). The third linear movement (7), which is linked in both ends (8), is used to get the correct angle of the blocks. The whole mechanism is linked in (14) to allow relative movements towards comer blocks (1) and spherical surfaces (2).

The control of the movements is taken care of by a computer receiving the requested shape of the beam. The signals are sent to a step motor (9), one for each movement, which by some type of transmission (10) runs a screw (11) which makes the linear movement.

To make sure that the setting is the decided one, a linear sensor (12) is installed, which checks the position and compare it with the position which has been reached by the motor.

## Claims

1. A device to limit radiation, especially the high energetic, consisting of a pre-collimator (15) for shaping square radiation areas and a final collimator (1-4, 8-12), the final collimator (1-4, 8-12) has collimation blocks (1) which are individually adjustable in two mutually perpendicular dimensions (5, 6),
**characterized in that** each block (1) further is individually rotatably supported in said final collimator (1-4, 8-12).

2. A device according to claim 1, wherein said collimator blocks (1) are situated between two spherical surfaces (2) and the edges of the blocks (1) are parallel to the radiation beam.

3. A device according to claim 1 or 2, wherein motors (9) are linked to said collimator blocks (1).

4. A device according to claim 1, 2 or 3, wherein the position of each collimator block (1) is controlled partly by a control motor, partly by a sensor (12).

5. A device according to any of claims 1-4, wherein the collimator block (1) material has high density, preferably lead and tungsten.

6. A device according to any of claims 1-5, wherein the number of collimator blocks (1) is at least four.

7. A device according to any of claims 1-6, wherein the whole collimator package can be rotated around the central line of the beam.

## Patentansprüche

1. Vorrichtung zum Begrenzen von Strahlung, insbesondere der hochenergetischen, die aus einem Vor-Kollimator (15) zum Formen quadratischer Strahlungsbereiche und einem Abschluss-Kollimator (1-4, 8-12) besteht, wobei der Abschluss-Kollimator (1-4, 8-12) Kollimationsblöcke (1) aufweist, die einzeln in zwei zueinander senkrechten Dimensionen (5, 6) verstellt werden können, **dadurch gekennzeichnet, dass** jeder Block (1) des Weiteren einzeln drehbar in dem Abschluss-Kollimator (1-4, 8-12) gelagert ist.

2. Vorrichtung nach Anspruch 1, wobei sich die Kollimatorblöcke (1) zwischen zwei kugelförmigen Flächen (2) befinden und die Ränder der Blöcke (1) parallel zu dem Strahlungsstrahl sind.

3. Vorrichtung nach Anspruch 1 oder 2, wobei Motoren (9) mit den Kollimatorblöcken (1) verbunden sind.

4. Vorrichtung nach Anspruch 1, 2 oder 3, wobei die Position jedes Kollimatorblocks (1) teilweise durch einen Steuermotor und teilweise durch einen Sensor (12) gesteuert wird.

5. Vorrichtung nach einem der Ansprüche 1-4, wobei das Material des Kollimatorblocks (1) hohe Dichte hat, d.h. vorzugsweise Blei und Wolfram ist.

6. Vorrichtung nach einem der Ansprüche 1-5, wobei die Anzahl von Kollimatorblöcken (1) wenigstens vier beträgt.

7. Vorrichtung nach einem der Ansprüche 1-6, wobei die gesamte Kollimatoreinheit um die Mittellinie des Strahls herum gedreht werden Kann.

## Revendications

1. Dispositif de limitation de rayonnement, en particulier de rayonnement à haute énergie, constitué d'un pré-collimateur (15) destiné à mettre des zones d'irradiation sous une forme carrée et un collimateur final (1 à 4, 8 à 12), le collimateur final (1 à 4, 8 à 12) comportant des blocs de collimation (1) qui sont ajustables individuellement suivant deux dimensions (5, 6) mutuellement perpendiculaires, **caractérisé en ce que** chaque bloc (1) est, en outre, supporté individuellement en rotation dans ledit collimateur final (1 à 4, 8 à 12).

2. Dispositif selon la revendication 1, dans lequel lesdits blocs de collimateur (1) sont situés entre deux surfaces sphériques (2) et les bords des blocs (1) sont parallèles au faisceau de rayonnement.

3. Dispositif selon la revendication 1 ou 2, dans lequel des moteurs (9) sont reliés auxdits blocs de collimateur (1).

4. Dispositif selon la revendication 1, 2 ou 3, dans lequel la position de chaque bloc de collimateur (1) est réglée en partie par un moteur de commande, en partie par un capteur (12).

5. Dispositif selon l'une quelconque des revendications 1 à 4, dans lequel le matériau des blocs de collimateur (1) présente une densité élevée, comprenant de préférence du plomb et du tungstène.

6. Dispositif selon l'une quelconque des revendications 1 à 5, dans lequel le nombre de blocs de collimateur (1) est d'au moins quatre.

7. Dispositif selon l'une quelconque des revendications 1 à 6, dans lequel l'ensemble de la structure de collimation peut être déplacé en rotation autour de l'axe central du faisceau.
